# EUROPEAN PATENT SPECIFICATION

(11) **EP 2 670 345 B1**
(45) Date of publication and mention of the grant of the patent: **01.02.2017**
(21) Application number: 12742286.3
(22) Date of filing: 03.02.2012
(51) Int. Cl.: A61F 2/01, A61B 17/221

(54) **VASCULAR AND BODILY DUCT TREATMENT DEVICES**
GEFÄSS- UND KÖRPERKANALBEHANDLUNGSVORRICHTUNGEN
DISPOSITIFS DE TRAITEMENT DE CONDUITS VASCULAIRES ET CORPORELS

(30) Priority: 04.02.2011 US 201113021364; 23.11.2011 US 201113303890
(43) Date of publication of application: 11.12.2013
(60) Divisional application: 16196958.9
(73) Proprietor: Concentric Medical, Inc., Mountain View, CA 94041 (US)
(72) Inventor: GRANDFIELD, Ryan M., Livermore, California 94550 (US); WILSON, Scott D., Redwood City, California 94061 (US); SANDERS, Elliot H., Palo Alto, California 94303 (US); MILLER, John H., Redwood City, California 94061 (US)
(74) Representative: ZBM Patents - Zea, Barlocci & Markvardsen
(86) International application number: PCT/US2012/023858
(87) International publication number: WO 2012/106657

(56) References cited:
- US-A1- 2004 236 368
- US-A1- 2007 198 051
- US-A1- 2008 208 244
- US-A1- 2010 161 034
- US-A1- 2011 009 950
- US-A1- 2011 009 950

## Description

### TECHNICAL FIELD

This application relates to devices and methods for treating the vasculature and other ducts within the body.

### BACKGROUND

Self-expanding prostheses, such as stents, covered stents, vascular grafts, flow diverters, and the like have been developed to treat ducts within the body. Many of the prostheses have been developed to treat blockages within the vasculature and also aneurysms that occur in the brain. What are needed are improved treatment methods and devices for treating the vasculature and other body ducts, such as, for example, aneurysms, stenoses, embolic obstructions, and the like.

US 2011/009950 discloses a duct obstruction retrieval device having strut members with different width dimensions.

### SUMMARY OF THE DISCLOSURE

In accordance with the invention, a duct obstruction retrieval device according to claim 1 is described. A preferred embodiment is described in claim 2.

### BRIEF DESCRIPTION OF THE DRAWINGS

Alternative implementations of the present disclosure are described herein with reference to the drawings wherein:
Figure 42A illustrates a two-dimensional plane view of clot retrieval devices according some implementations.
Figure 42B illustrates an enlarged two-dimensional plane view of the proximal tapered end portion of the retriever device depicted in Figure 45 A.
Figure 43 illustrates a two-dimensional plane view of a proximal-most cell structure according some implementations.
Figure 44 illustrates a two-dimensional plane view of a proximal-most cell structure according some implementations.

### DETAILED DESCRIPTION

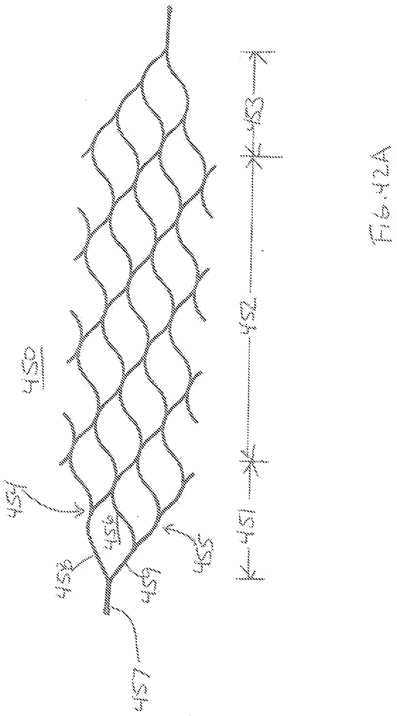

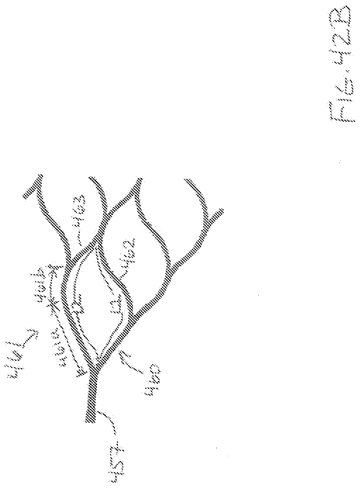

Figure 42A is a two dimensional view of a duct obstruction retrieval device 450 according to an implementation. The retrieval device 450 comprises an expandable member that has a proximal tapered end portion 451, a cylindrical, main body portion 452 and a distal tapered end portion 453. The outer-most cell structures in the proximal tapered end portion have outer wall segments that form first and second rail segments 454 and 455, respectively. Each of the rail segments 454 and 455 extend from a proximal -most end of the expandable member to a position at or near the proximal end of the cylindrical main body portion 452. In the implementation of Figure 42, each of the rail segments 454 and 455 are undulating. A proximal antenna 457 extends proximally from a proximal-most cell structure 456.

The proximal-most cell structure 456, as shown in greater detail in Figure 42B, comprises first and second outer struts 460 and 461, respectively, and first and second inner struts 462 and 463, respectively. As shown in the layout of Figure 42B, the first outer strut 460 and a first portion 461 a of the second outer strut 46.1 are straight in the two dimensional layout while the first inner strut 462, second inner strut 463 and the second portion 461 b of strut 461 are curvilinear in the two dimensional layout. In the manufactured, three dimensional configuration the first outer strut 460 and the first portion 461 a of the second outer strut 461 are curved and devoid of undulations. As a result of being oriented at the proximal end of the expandable member and being coextensive to the proximal antenna, the straight strut segments of the proximal-most cell structure 456 enhance the pushability of the retrieval device 450 as it is delivered, through the anatomy of a patient as compared to retrieval devices having proximal-most cell structure with only curved struts in the two dimensional layout.

In some implementations, the total length of struts 460 and 462 (LI) and the total length of struts 461 and 463 (L2) are substantially the same in order to promote a nesting of the struts when the expandable member transitions from the expanded state to the unexpanded state. According to some implementations the difference in length between LI and L2 is less than 5.0%, while in other implementations the difference in length between LI and L2 is less than 1.0%.

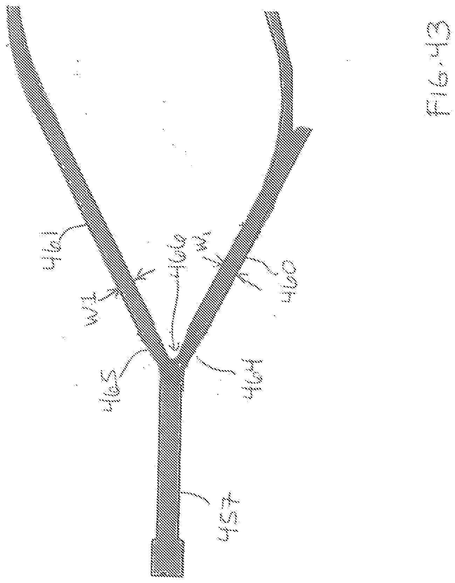

Figure 43 illustrates a variation of the proximal-most cell structure 456. As depicted, each of struts 460 and 461 have an area of reduced width 464 and 465, respectively, that are located adjacent their junction 466 with the proximal antenna 457. The inclusion of the reduced width areas 464 and 465 locally enhances the proximal-most cell structure's ability to collapse by reducing the amount of force needed to initiate and effectuate the collapse. Thus, for example, when the retrieval device 450 is first introduced into an introducer sheath for placement within a delivery catheter or is withdrawn into a delivery catheter after the expandable member has deployed inside a patient, the areas of reduced width 464 and 465 cause the shuts 460 and 461 to be more easily folded in the area of the junction 466 with less force than would otherwise be required absent the areas of reduced width. This makes the retrieval device 450 more manageable when being handled by healthcare professionals when the retrieval device 450 is being introduced into the delivery catheter for the first time, thus reducing the likelihood of the retrieval device being damaged during the introduction process. As previously discussed, after the retrieval device 450 has been introduced and expanded inside the duct of a patient there may be occasions when the retrieval device is proximally withdrawn back into the delivery catheter. This may occur, for example, upon the retrieval device being improperly placed in the duct or upon the completion of a retrieval procedure. In each of these instances because less force is required to collapse the expandable member of the retrieval device several advantages are realized. One advantage is that it reduces the likelihood of the retrieval device 450 acting upon the delivery catheter in a manner that would cause an inadvertent displacement of the delivery catheter within the duct of the patient. Another advantage is that it reduces the likelihood of excessive force being applied at the attachment between the proximal antenna 457 and the elongate wire (e.g. elongate wire 40 shown in Figure 1 A) that would result in a failure at the junction.

In the implementation of Figure 43 the areas of reduced width 464 and 465 comprise tapers. In other implementations the areas of reduced width are denoted by a stepped reduction in strut width. The amount by which the width is reduced in areas 464 and 465 will vary according to the nominal widths of struts 460 and 461. In any event, it is important that the amount of width reduction is consistent with the radial force and structural integrity requirements of the expandable member. It has been discovered that a reduction of width in the as-cut manufactured state of between about 5.0% and about 20.0% is suitable for struts having a nominal width of between about 0.0057 inches and about 0.0027 inches, (between about 0.145 mm and about 0.068 mm), with a preferable range being between about 10.0% and about 20.0% in width reduction. In one implementation the width dimension W1 of struts 460 and 461 is about 0.0053 inches (about 0.135 mm) with the minimum width dimension of the areas of reduced width being 0.0047 inches (0.12 mm). In another implementation the width dimension W1 of strut s 460 and 461 is about 0.0057 inches (about 0.145 mm) with the minimum width dimension of the areas of reduced width being 0.0046 inches (0.117 mm).

In some implementations the as-cut width dimensions of struts 460 and 461 are different, with the width dimension of their respective areas of reduced width 464 and 465 also being different. For example, in one implementation strut 460 has a width dimension of about 0.0050 inches (about 0.127 mm), strut 461 has a width dimension of about 0.0057 inches (about 0.145 mm), and areas of reduce width 464 and 465 have width dimensions of about 0.0042 inches (about 0.107 mm) and about 0,0046 inches (about 0.117 mm), respectively.

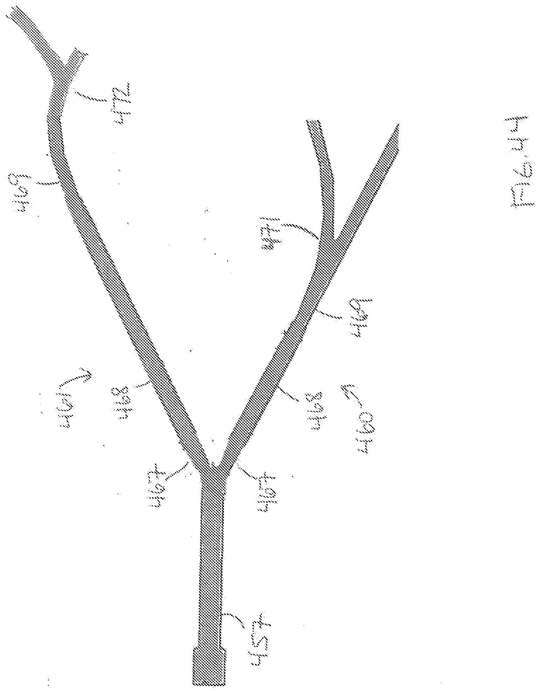

Figure 44 shows another variation of the proximal-most cell structure 457 wherein outer struts 460 and 461 comprise a proximal section 467, a midsection 468 and a distal section 469. Because the width dimensions of the outer struts 460 and 461 of the proximal-most cell structure 456 are generally made greater than most of struts in the remaining portion of the retrieval device 450 for the purpose of enhancing the pushability of the expandable member, the bulk of material at the junctures 471 and 472 located at the distal end of the struts may impede the expandable member's ability to collapse. For this reason, in the implementation of Figure 44 the distal sections 469 have a reduced width dimension in order to reduce the amount of material occupying the juncture regions 471 and 472. Although Figure 44 also shows the proximal sections 467 having a reduced width dimension (similar to that described above), in some implementations this is not the case. In a manner described above, the sections of reduced width may comprise tapers and/or steps.

Another advantage of the implementation depicted in Figure 44 is that the midsection 468 of struts 460 and 461 ma}' be provided with a sufficient width to enhance the visibility of the device under fluoroscopy without materially impacting the ability of the proximal end of the proximal tapered region 451 to collapse or to otherwise assume its unexpanded state. According to one implementation the width dimension of the strut midsections 468 is about 0.0053 inches (about 0.135 mm) and the minimum width dimension of the proximal and distal sections 467 and 469 being 0.0047 inches (0.119 mm) and 0.0041 inches (0.104 mm), respectively. As with some of the Figure 43 implementations, in some Figure 44 implementations the width dimensions of struts 460 and 461 are different, with the width dimension of one or more of their respective proximal sections, midsections and distal sections being different.

While the above description contains many specifics, those specifics should not be construed as limitations on the scope of the disclosure, but merely as exemplifications of preferred embodiments thereof. For example, dimensions other than those listed above are contemplated. For example, retrieval devices having expanded diameters of anywhere between 1.0 and 100.0 millimeters and lengths of up to 5.0 to 10.0 centimeters are contemplated. Moreover, it is appreciated that many of the features disclosed herein are interchangeable among the various implementations. Those skilled in the art will envision many other possible variations that are within the scope and spirit of the disclosure. Further, it is to be appreciated that the delivery of a vascular treatment device of the implementations disclosed herein is achievable with the use of a catheter, a sheath or any other device that is capable of carrying the device with the expandable member in a compressed state to the treatment site and which permits the subsequent deployment of the expandable member at a vascular treatment site. The vascular treatment site may be (1) at the neck of an aneurysm for diverting flow and/or facilitating the placement of coils or other like structures within the sack of an aneurysm, (2) at the site of an embolic obstruction with a purpose of removing the embolic obstruction, (3) at the site of a stenosis with a purpose of dilating the stenosis to increase blood flow through the vascular, etc.

## Claims

1. A duct obstruction retrieval device comprising: an expandable member having a proximal tapered end portion (451) with cell structures, the cell structures extending less than circumferentially around a longitudinal axis of the expandable member, the outer-most cell structures in the proximal tapered end portion (451) having outer wall segments that form first and second peripheral rail segments (454, 455), the proximal tapered end portion having a proximal-most cell structure (456), the proximal-most cell structure (456) comprising first and second outer struts (460, 461) that extend distally from a proximal antenna (457), in a two-dimensional layout of the expandable member at least a portion of each of the first and second outer struts (460, 461) comprise a straight segment, each of the straight segments being coextensive with the proximal antenna (457), the proximal-most cell structure (456) comprising the first outer strut (460), the second outer strut (461), a first inner strut (462) and a second inner strut (463), the first inner strut (462) extending distally from the first outer strut (460), the second inner strut (463) extending distally from the second outer strut (461), in the two-dimensional layout of the expandable member all or substantially all of the first and second inner struts (462, 463) are curvilinear;
**characterized in that** in the two-dimensional layout of the expandable member the first outer strut (460) comprises a first proximal portion (464) adjacent to the proximal antenna (457) that is straight and the second outer strut (461) comprises a second proximal portion (465) adjacent to the proximal antenna (457) that is straight, each of the first and second proximal portions (465, 464) having a width dimension less than the width dimension of the remainder of the first and second outer struts (460, 461), respectively.

2. A duct obstruction retrieval device according to claim 1, wherein the first and second proximal portions (464, 465) comprise tapers.

## Patentansprüche

1. Eine Vorrichtung zur Röhrenverschlussrückgewinnung umfassend: ein expandierbares Element, das einen proximalen verjüngten Endteil (451) mit Zellstrukturen hat, wobei sich die Zellstrukturen weniger als umlaufend um eine Längsachse des expandierbaren Elements herum erstrecken, wobei die äußersten Zellstrukturen im proximalen verjüngten Endteil (451) äußere Wandabschnitte haben, die erste und zweite Umfangsschienabschnitte (454, 455) bilden, wobei der proximale verjüngte Endteil eine proximalste Zellstruktur (456) hat, wobei die proximalste Zellstruktur (456) erste und zweite äußere Streben (460, 461) hat, die sich distal von einer proximalen Antenne (457) heraus erstrecken, wobei in einer zweidimensionalen Anordnung des expandierbaren Elements mindestens ein Teil von jeder der ersten und zweiten äußeren Streben (460, 461) einen geraden Abschnitt umfasst, wobei jeder gerade Abschnitt koextensiv mit der proximalen Antenne (457) ist, wobei die proximalste Zellstruktur (456) die erste äußere Strebe (460), die zweite äußere Strebe (461), eine erste innere Strebe (462) und eine zweite innere Strebe (463) umfasst, wobei sich die erste innere Strebe (462) distal von der ersten äußeren Strebe (460) hinaus erstreckt, die zweite innere Strebe (463) sich distal von der zweiten äußeren Strebe (461) hinaus erstreckt, wobei in der zweidimensionalen Anordnung des expandierbares Elements alle oder im Wesentlichen alle ersten und zweiten inneren Streben (462, 463) krummlinig sind;
**dadurch gekennzeichnet, dass** in der zweidimensionalen Anordnung des expandierbaren Elements die erste äußere Strebe (460) einen an die proximale Antenne (457) angrenzenden ersten proximalen Teil (464) umfasst, der gerade ist und die zweite äußere Strebe (461) einen an die proximale Antenne (457) angrenzenden zweiten proximalen Teil (465) umfasst, der gerade ist, wobei jeder der ersten und zweiten proximalen Teile (465, 464) ein Breitenmaß hat, das jeweils geringer als das Breitenmaß der weiteren ersten und zweiten äußeren Streben (460, 461) ist.

2. Eine Vorrichtung zur Röhrenverschlussrückgewinnung nach Anspruch 1, wobei der erste und der zweite proximale Teil (464, 465) Verjüngungen umfassen.

## Revendications

1. Un dispositif de récupération d'obstructions de conduit comprenant: un élément expansible ayant une partie d'extrémité effilée proximale (451) avec des structures cellulaires, s'étendant les structures cellulaires moins que circonférentiellement autour d'un axe longitudinal de l'élément expansible, ayant les structures cellulaires ultrapériphériques dans la partie d'extrémité effilée proximale (451) des segments de paroi extérieurs qui forment des segments de rail périphériques premiers et deuxièmes (454, 455), ayant la partie d'extrémité effilée proximale une structure cellulaire ultraproximale (456), comprenant la structure cellulaire ultraproximale (456) des entretoises extérieures première et deuxième (460, 461) qui s'étendent distalement à partir d'une antenne proximale (457), dans une disposition bidimensionale de l'élément expansible au moins une partie de chacune des entretoises extérieures première et deuxième (460, 461) comprennent un segment droit, étant chacun des segments droits coextensif avec l'antenne proximale (457), comprenant la structure cellulaire ultraproximale (456) la première entretoise extérieure (460), la deuxième entretoise extérieure (461), une première entretoise intérieure (462) et une deuxième entretoise intérieure (463), s'étendant la première entretoise intérieure (462) distalement à partir de la première entretoise extérieure (460), s'étendant la deuxième entretoise intérieure (463) distalement à partir de la deuxième entretoise extérieure (461), dans la disposition bidimensionale de l'élément expansible toutes ou essentiellement toutes les entretoises intérieures première et deuxième (462, 463) sont curvilignes;
**caractérisé en ce que** dans la disposition bidimensionale de l'élément expansible la première entretoise extérieure (460) comprend une première partie proximale (464) adjacente à l'antenne proximale (457) qui est droite et la deuxième entretoise extérieure (461) comprend une deuxième partie proximale (465) adjacente à l'antenne proximale (457) qui est droite, ayant chacune des parties proximales première et deuxième (465, 464) une dimension en largeur inférieure à la dimension en largeur du reste des entretoises extérieures première et deuxième (460, 461), respectivement.

2. Un dispositif de récupération d'obstructions de conduit selon la revendication 1, dans lequel les parties proximales première et deuxième (464, 465) comprennent des effilements.
